# EUROPEAN PATENT APPLICATION

(11) **EP 3 912 578 A1**
(43) Date of publication of application: **24.11.2021**
(21) Application number: 21174452.9
(22) Date of filing: 18.05.2021
(51) Int. Cl.: A61B 18/12, A61B 90/00, A61B 18/14, A61B 18/00

(54) **ESOPHAGEAL-TISSUE TEMPERATURE MONITORING**

(30) Priority: 19.05.2020 US 202016877737
(71) Applicant: Biosense Webster (Israel) Ltd, Yokneam, 2066717 (IL)
(72) Inventor: DEKEL, Zvi, 2066717 Yokneam (IL); SUSEL, Pesach, 2066717 Yokneam (IL)
(74) Representative: Carpmaels & Ransford LLP

(57) **Abstract**

An apparatus includes a camera and a processor. The camera is configured to capture images of a display of a temperature measurement system that displays a tissue temperature. The processor is configured to analyze the captured images to extract a numerical value of the tissue temperature displayed by the temperature measurement system, and initiate an action responsively to the extracted numerical value.

## Description

### FIELD OF THE INVENTION

The present invention relates generally to cardiac ablation, and specifically to monitoring esophageal-tissue temperature during ablation.

### BACKGROUND OF THE INVENTION

Techniques for sensing esophageal tissue temperature during cardiac ablation were previously reported in the patent literature. For example, U.S. Patent 9,033,968 describes a method and system for increasing safety of cardiac ablation procedures using a computer-based system that monitors the esophageal temperature, the system comprising an esophageal temperature sensing means, typically on a probe inserted into the esophagus. During atrial fibrillation ablations, based on a pre-determined increase in esophageal temperature, the computer-based system activates different levels of alarm(s), and/or initiates ablation energy interrupt based on pre-defined programmed values.

As another example, U.S. Patent 8,971,997 describes an endoscopic infrared fiber-optic device able to monitor esophageal temperature during an ablation/cryoablation procedure over a volume of interest to sense whether the temperature is too high or too low. The device may include a plurality of optical fibers each with a wide-angle lens collectively disposed circumferentially and longitudinally to cover the volume of interest, as the particular region over which undesirable temperature may not be known beforehand. In other examples, the device may include an embedded array of infrared sensors extending sufficiently to encompass a volume of interest. The device may be used as part of a feedback control to regulate and stop operation of the ablation/cryoablation procedure to prevent vessel damage.

U.S. Patent Application Publication 2006/0106375 describes devices, systems and methods for the ablation of tissue and treatment of cardiac arrhythmia. An ablation system includes an ablation catheter that has an array of ablation elements and a location element, an esophageal probe also including a location element, and an interface unit that provides energy to the ablation catheter. The distance between the location elements, determined by calculating means of the system, can be used by the system to set or modify one or more system parameters. To avoid damage to the esophagus, a system of the present invention preferably uses a temperature threshold for a temperature detected using a thermocouple on the esophageal probe.

### SUMMARY OF THE INVENTION

An embodiment of the present invention that is described hereinafter provides an apparatus including a camera and a processor. The camera is configured to capture images of a display of a temperature measurement system that displays a tissue temperature. The processor is configured to analyze the captured images to extract a numerical value of the tissue temperature displayed by the temperature measurement system, and initiate an action responsively to the extracted numerical value.

In some embodiments, the tissue temperature includes a temperature of an esophagus of a patient undergoing a cardiac ablation procedure, and the processor is configured to initiate termination of the cardiac ablation procedure.

In some embodiments, the processor is configured to provide the extracted numerical value of the tissue temperature for display by another system.

In an embodiment, the processor is configured to analyze the captured images by performing image processing over a region of interest (ROI) in the captured images.

In an embodiment, the temperature measurement system displays the tissue temperature using alphanumeric characters, and the processor is configured to extract the numerical value by recognizing the alphanumeric characters in the images. In another embodiment, the temperature measurement system displays the tissue temperature using an analog graphic display, and the processor is configured to extract the numerical value by analyzing the analog graphic display in the images.

In some embodiments, the processor is configured to issue a triggering signal in response to the extracted temperature deviating from a prespecified limit.

In some embodiments, the processor is further configured to calculate a rate of change of the tissue temperature, and to initiate the action in response to the calculated rate of change.

In an embodiment, the processor is configured to issue a triggering signal in response to the rate of change deviating from a prespecified limit.

In another embodiment, the processor is included in an RF generator and is configured to initiate the action by changing an output power of the RF generator output power.

There is additionally provided, in accordance with an embodiment of the present invention, a method including, using a camera, capturing images of a display of a temperature measurement system that displays a tissue temperature. The captured images are analyzed to extract a numerical value of the tissue temperature displayed by the temperature measurement system. An action is initiated responsively to the extracted numerical value.

### BRIEF DESCRIPTION OF THE DRAWINGS

The present invention will be more fully understood from the following detailed description of the embodiments thereof, taken together with the drawings in which:
Fig. 1 is a schematic, pictorial illustration of a catheter-based cardiac radiofrequency (RF) ablation system comprising an automated esophageal-tissue monitoring apparatus, in accordance with an exemplary embodiment of the present invention;
Fig. 2 is a schematic, pictorial illustration showing the ablation balloon of Fig. 1 positioned at an ostium of a left atrium in the vicinity of the esophagus, in accordance with an exemplary embodiment of the present invention; and
Fig. 3 is a flow chart that schematically illustrates a cardiac ablation procedure aided by the automated esophageal tissue monitoring apparatus of Fig. 1, in accordance with an exemplary embodiment of the present invention.

### DETAILED DESCRIPTION OF EMBODIMENTS

### OVERVIEW

An anatomic relationship between target tissue undergoing ablation and nearby unrelated tissue can cause problems in invasive ablation of the target tissue, such as unintentional overheating of the nearby unrelated tissue. Specifically, for cardiac ablation, the esophagus lies posterior to the left atrium and leads a variable course relative to the left atrium, adjacent to the right or left pulmonary vein or the posterior wall of the heart. Hence, there is a potential risk of esophageal damage due to, in cases of radiofrequency (RF) or laser ablation, the high temperatures caused when ablation is performed anywhere in the posterior left atrium. Similarly, cryoablation may potentially cause collateral damage by accidently cryoablating an esophageal-tissue.

To prevent damage to the esophagus, a third-party system (i.e., a system distinct from the ablation system) can be used for esophagus temperature monitoring. Such a system typically provides a numerical display of the esophagus temperature, or uses other types of graphical means to display the temperature, such as an analog scale or analog-like display. The physician performing the ablation, or an assistant, can monitor the third-party system display while performing the ablation. If the user reads from the third-party system an indication of an esophageal-tissue temperature being outside an allowable range or expectation of such event to occur (e.g., rate of increase of temperature estimated by the user to be too high), the user (e.g., the physician) may abort the ablation, to prevent damage to the esophagus.

However, since the third-party monitoring system is detached from the ablation system, human intervention is relied upon to control the ablation responsively to indications from the third-party system. Such human involvement may be slow or erroneous, and therefore inadvertent esophagus damage may occur (e.g., due to accidental overheating or overcooling, depending on the ablation method).

Exemplary embodiments of the present invention that are described hereinafter provide improved methods and systems for monitoring esophageal temperature and controlling ablation procedures accordingly. In a disclosed exemplary embodiment, an apparatus comprises a camera used to observe and acquire an image of the third-party display that includes a region of interest (ROI) comprising displayed esophageal temperature. A processor comprised/used in the apparatus analyzes the ROI, using image processing techniques, to identify the displayed esophageal temperature (e.g., to extract a numerical temperature value included in the ROI).

Subsequently, the processor initiates an action responsively to the extracted numerical value. For example, the processor may check the identified temperature to determine if the temperature of the unrelated tissue deviates beyond prespecified temperature limits, or if the rate of change of the temperature deviates beyond a prespecified allowable rate (i.e., temperature and/or rate of change of the temperature deviating from a prespecified limit).

For example, in case of RF ablation, the processor checks if a temperature threshold has been exceeded, or if the rate of increase of temperature is too high. In case of cryoablation, the processor checks if the temperature fell below an allowed value or the rate of fall of temperature is too high.

In some exemplary embodiments, in response to determining that a temperature deviation is occurring, the processor outputs a triggering signal. In case of RF ablation, the triggering signal is received by an RF generator control unit, which in turn terminates the ablation responsively to receiving the triggering signal. In other exemplary embodiments, the processor is comprised in the RF generator and initiates an action comprising changing a setting of the RF generator, including terminating the ablation by shutting off or minimizing the power outputted by the RF generator.

In some exemplary embodiments, the disclosed monitoring apparatus provides the identified (e.g., extracted) temperature, and optionally its calculated rate of change, for display by the ablation system. That way, the physician is better aware in real-time to risks of collateral damage from the ablation. In addition, in case the ablation is terminated automatically, the physician may be informed by various audiovisual means, such as changing the ablation display colors and/or by using sounding alerts included in the ablation system.

Typically, the processor is programmed in software containing a particular algorithm that enables the processor to conduct each of the processor related steps and functions outlined above.

By providing a monitoring apparatus capable of, during invasive ablation of an internal organ such as the heart, identifying a thermal hazard to nearby tissue, such as esophageal-tissue, and responsively automatically terminating the ablation, ablative treatments may be made safer.

### ESOPHAGEAL-TISSUE TEMPERATURE-MONITORING TO TERMINATE CARDIAC RF ABLATION AUTOMATICALLY

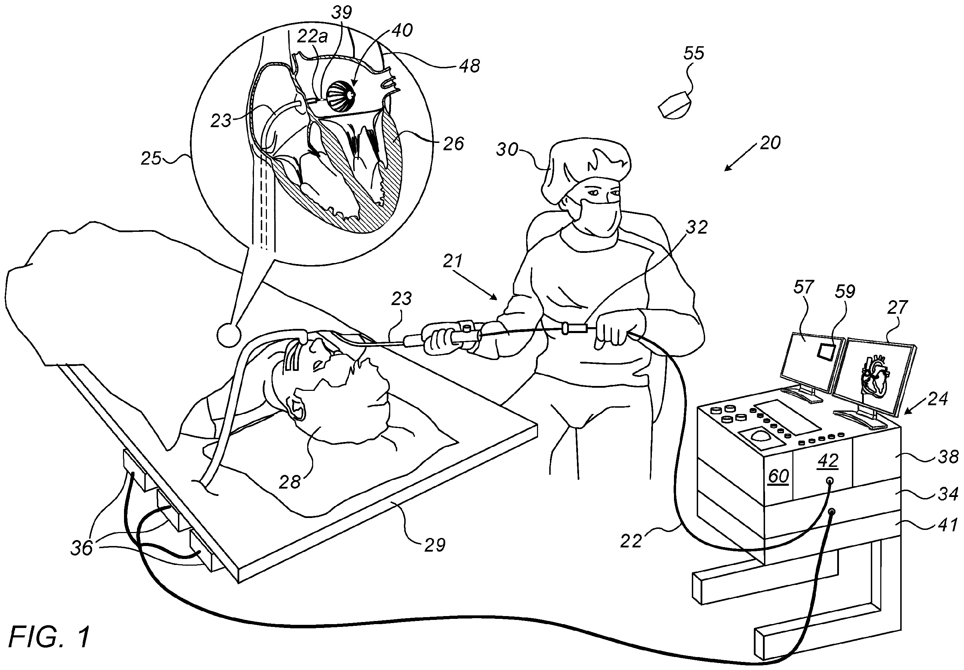

Fig. 1 is a schematic, pictorial illustration of a catheter-based cardiac radiofrequency (RF) ablation system 20 comprising an automated esophageal-tissue monitoring apparatus, in accordance with an exemplary embodiment of the present invention. System 20 comprises a catheter 21, wherein, as seen in inset 25, a distal end 22a of shaft 22 of catheter 21 is inserted through a sheath 23 into a heart 26 of a patient 28 lying on a table 29. As further shown in inset 25, distal end 22a comprises a magnetic sensor 39, contained within distal end 22a just proximally to a radiofrequency ablative balloon 40. Sensor 39 is used by system 20 to navigate the catheter to a target position. However, the disclosed monitoring technique can be applied with any other navigational solution, such as based on electrical impedance signals, or even be applied with catheters that do not include or are not positioned using a navigational means.

While the shown exemplary embodiment uses a balloon ablation catheter, the disclosed monitoring technique can be used with any invasive ablation device, and in particular with any type of ablation catheter.

The proximal end of catheter 21 is connected to a control console 24. In the exemplary embodiment described herein, catheter 21 may be used for any suitable therapeutic and/or diagnostic purpose, such as electrical ablation using an RF generator 42 comprised in console 24 and/or sensing of tissue in heart 26. However, for clarity, the disclosed technique is focused on monitoring a therapeutic procedure.

During navigation of distal end 22a in heart 26, console 24 receives signals from magnetic sensor 39 in response to magnetic fields from external field generators 36, for example, for the purpose of measuring the position of ablation balloon 40 in the heart 26 and, optionally, presenting the tracked position on a display 27. Magnetic field generators 36 are placed at known positions external to patient 28, e.g., below patient table 29. Console 24 also comprises a driver circuit 34, configured to drive magnetic field generators 36.

In an exemplary embodiment, position signals received from position sensor 39 are indicative of the position of ablation balloon 40 in the coordinate system of position tracking and ablation system 20. The method of position sensing using external magnetic fields is implemented in various medical applications, for example, in the CARTO™ system, produced by Biosense-Webster Inc. (Irvine, California), and is described in detail in U.S. Patents 5,391,199, 6,690, 963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1, whose disclosures are all incorporated herein by reference.

Physician 30 navigates the distal end of shaft 22 to a target location in heart 26 by manipulating shaft 22 using a manipulator 32 near the proximal end of the catheter and/or deflection from the sheath 23. The balloon 40 may be proximate the esophagus 48 as explained in greater detail subsequently. During the insertion of shaft 22, balloon 40 is maintained in a collapsed configuration by sheath 23. By containing balloon 40 in a collapsed configuration, sheath 23 also serves to minimize vascular trauma along the way to target location.

Control console 24 comprises a processor 41, typically a general-purpose computer, with suitable front end and interface circuits 38 for receiving signals from catheter 21, as well as for applying ablative treatment via catheter 21 in heart 26 and for controlling the other components of system 20.

As seen in Fig. 1, a camera 55 is positioned to acquire images of a third-party monitor 57 in real time, wherein monitor 57 displays esophageal-tissue temperature information inside an ROI 59, during the ongoing ablation. In the illustrated exemplary embodiment, the acquired images are sent, e.g., wirelessly, to processor 41, which uses an algorithm to analyze the images, using imaging processing techniques, so as to identify in ROI 59 of the images an esophageal tissue temperature, and subsequently to calculate a rate of change of the temperature. In other embodiments, however, camera 55 is connected directly, either with a cable or wirelessly (e.g., by a Bluetooth link), to a control circuitry of RF generator 42.

In an exemplary embodiment, processor 41 is configured to compare the temperature to a threshold value and compare the rate of change of the temperature to an allowable rate, both of which being prespecified. If the temperature exceeds the threshold and/or exceeds the allowable rate, processor 41 triggers a control unit 60 of RF generator 42 of system 20 to responsively terminate ablation, for example by control unit 60 switching a relay on an RF power line. In other exemplary embodiments, however, an indication from camera 55 may be directly transmitted to and trigger control unit 60 of RF generator 42.

Furthermore, processor 41 shows on display 27 (e.g., CARTO ablation system display) the extracted esophageal-tissue temperature and the calculated rate of change of the temperature and informs the physician by various means, such as changing display colors and sounding alerts, that the ablation had to be terminated automatically, due to one of the aforementioned thermal hazards.

Processor 41 typically comprises a general-purpose computer with software programmed to carry out the functions described herein. The software may be downloaded to the computer in electronic form, over a network, for example, or it may, alternatively or additionally, be provided and/or stored on non-transitory tangible media, such as magnetic, optical, or electronic memory.

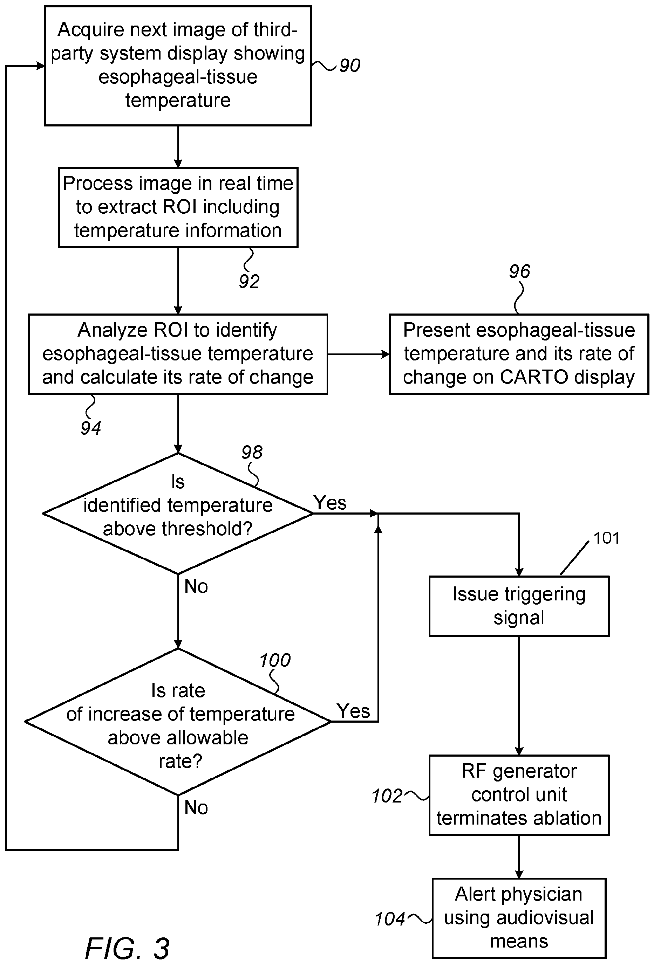

In particular, processor 41 runs a dedicated algorithm as disclosed herein, including in Fig. 3, which enables processor 41 to perform the disclosed steps, as further described below.

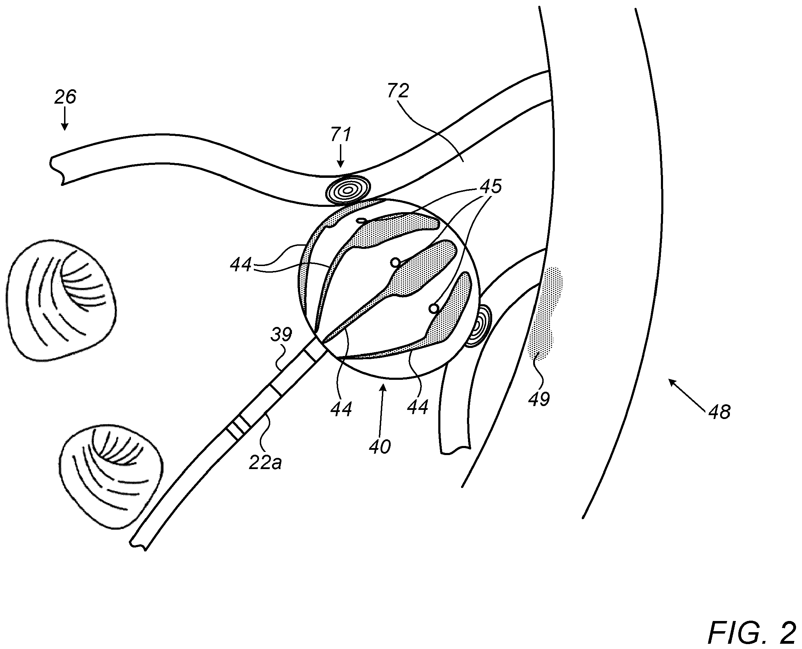

Fig. 2 is a schematic, pictorial illustration showing ablation balloon 40 of Fig. 1 positioned at an ostium 71 of a pulmonary vein 72 in the left atrium of heart 26 in vicinity of esophagus 48, in accordance with an exemplary embodiment of the present invention. Balloon 40 comprises multiple electrodes 44 that are distributed around its outer surface. As seen, some of electrodes 44 face the wall of esophagus 48 and are at close proximity to the wall tissue. Balloon 40 also comprises temperature sensors 45, wherein each temperature sensor 45 is in proximity to an electrode 44.

As shown in Fig. 2, a portion of esophagus 48, an esophageal wall tissue 49, is particularly vulnerable to being overheated during an ablation. Typically, esophageal wall tissue 49 at risk comprises a segment of the esophageal wall facing the posterior side of ostium 71. Thus, in some exemplary embodiments, to ease the work of the physician, the disclosed apparatus presents on CARTO® display 27 an anatomy similar to the anatomy shown in Fig. 2 with the identified temperature of esophageal wall tissue at risk 49 overlaid on the anatomy.

The example configuration shown in Fig. 2 is chosen purely for the sake of conceptual clarity. The disclosed techniques may similarly be applied using other system components and settings. For example, system 20 may comprise other sorts of ablation devices, such as a circular multi-electrode catheter (e.g., the Lasso@ catheter made by Biosense Webster Inc.) or a multi-branch multi-electrode catheter (e.g., PentaRay® made by Biosense Webster Inc.).

As another example, the disclosed treatment method may utilize devices based on laser ablative power, such as a laser ablation balloon that is fitted to the catheter distal end. Laser power would then be terminated by a control unit analogous to control unit 60 to avoid causing collateral thermal damage.

Fig. 3 is a flow chart that schematically illustrates a cardiac ablation procedure aided by the automated esophageal tissue monitoring apparatus of Fig. 1, in accordance with an exemplary embodiment of the present invention. The procedure begins at an image acquisition step 90, in which camera 55 acquires (e.g., captures) video or still images of a display of a third-party temperature measurement system that show a tissue temperature, such as an esophageal-tissue temperature measured during cardiac ablation.

Processor 41 receives the images and, using an algorithm, extracts an ROI of the image that contains the temperature information (e.g., contains a numerical value or an analog scale), at an image ROI extraction step 92. Next, processor 41 applies image processing to the extracted ROI to identify the temperature value (e.g., performs optical character recognition (OCR) if it is a numerical display, or other image processing if it is some analog scale or analog-like display). For example, processor 41 identifies, from the image ROI, a temperature of an esophageal tissue 49 at risk, as well as calculates a rate of change of the temperature, at a temperature identification step 94.

At a temperature value outputting step 96, processor 41 outputs a current (e.g., real time) identified temperature and calculated rate of change of the temperature of esophageal tissue 49 to CARTO® display 27. The display may be alphanumeric and/or an analog graphical information which can be overlaid on a presented anatomy.

Processor 41 compares the temperature to a threshold value and compares the rate of change of the temperature to an allowable rate, which are both prespecified. At a temperature checking step 98, if temperature exceeds threshold processor 41 triggers control unit 60, by issuing a triggering signal at a triggering step 101, to terminate the ablation. In response to the received triggering signal, control unit 60 terminates the ablation, at an ablation termination step 102. Similarly, if the rate of change (typically, an increase) of the temperature is checked (100) and found above an allowable rate, processor 41 triggers control unit 60 to terminate the ablation.

Finally, at an alerting step 104, the physician 30 is alerted by audiovisual means, as described above, that the system has automatically terminated ablation.

If, on the other hand, checking steps 98 and 100 find that both temperature and its rate of change are within limits, the process returns to thermal acquisition step 90.

The example flow chart shown in Fig. 3 is shown here purely for the sake of conceptual clarity. In alternative exemplary embodiments, the disclosed technique may use different and/or additional steps, such as, for example, monitoring each electrode 44 temperature using the corresponding temperature sensor 45 and modifying treatment accordingly.

Although the embodiments shown in the figures relate to a specific organ and type of treatment, the principles of the invention may be applied in preventing collateral damage to nearby organs in other organs, such as to kidneys and liver.

It will be thus appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

### ASPECTS OF THE INVENTION

1. A method for monitoring esophageal temperature and controlling ablation, the method comprising:
   using a camera, capturing images of a display of a temperature measurement system that displays a tissue temperature;
   analyzing the captured images to extract a numerical value of the tissue temperature displayed by the temperature measurement system; and
   initiating an action responsively to the extracted numerical value.
2. The method according to aspect 1, wherein the tissue temperature comprises a temperature of an esophagus of a patient undergoing a cardiac ablation procedure, and wherein initiating the action comprises initiating termination of the cardiac ablation procedure.
3. The method according to aspect 1, and comprising providing the extracted numerical value of the tissue temperature for display by another system.
4. The method according to aspect 1, wherein analyzing the captured images comprises performing image processing over a region of interest (ROI) in the captured images.
5. The method according to aspect 1, wherein the temperature measurement system displays the tissue temperature using alphanumeric characters, and wherein extracting the numerical value comprises recognizing the alphanumeric characters in the images.
6. The method according to aspect 1, wherein the temperature measurement system displays the tissue temperature using an analog graphic display, and wherein extracting the numerical value comprises analyzing the analog graphic display in the images.
7. The method according to aspect 1, wherein initiating the action comprises issuing a triggering signal in response to the extracted temperature deviating from a prespecified limit.
8. The method according to aspect 1, and comprising calculating a rate of change of the tissue temperature, wherein initiating the action comprises initiating the action in response to the calculated rate of change.
9. The method according to aspect 8, wherein initiating the action comprises issuing a triggering signal in response to the rate of change deviating from a prespecified limit.
10. The method according to aspect 1, wherein initiating the action comprises changing an output power of an RF generator.

## Claims

1. An apparatus for monitoring esophageal temperature and controlling ablation, the apparatus comprising:
a camera configured to capture images of a display of a temperature measurement system that displays a tissue temperature; and
a processor, which is configured to analyze the captured images to extract a numerical value of the tissue temperature displayed by the temperature measurement system, and initiate an action responsively to the extracted numerical value.

2. The apparatus according to claim 1, wherein the tissue temperature comprises a temperature of an esophagus of a patient undergoing a cardiac ablation procedure, and wherein the processor is configured to initiate termination of the cardiac ablation procedure.

3. The apparatus according to claim 1, wherein the processor is configured to provide the extracted numerical value of the tissue temperature for display by another system.

4. The apparatus according to claim 1, wherein the processor is configured to analyze the captured images by performing image processing over a region of interest (ROI) in the captured images.

5. The apparatus according to claim 1, wherein the temperature measurement system displays the tissue temperature using alphanumeric characters, and wherein the processor is configured to extract the numerical value by recognizing the alphanumeric characters in the images.

6. The apparatus according to claim 1, wherein the temperature measurement system displays the tissue temperature using an analog graphic display, and wherein the processor is configured to extract the numerical value by analyzing the analog graphic display in the images.

7. The apparatus according to claim 1, wherein the processor is configured to issue a triggering signal in response to the extracted temperature deviating from a prespecified limit.

8. The apparatus according to claim 1, wherein the processor is further configured to calculate a rate of change of the tissue temperature, and to initiate the action in response to the calculated rate of change.

9. The apparatus according to claim 8, wherein the processor is configured to issue a triggering signal in response to the rate of change deviating from a prespecified limit.

10. The apparatus according to claim 1, wherein the processor is comprised in an RF generator, and is configured to initiate the action by changing an output power of the RF generator.
